# EUROPÄISCHE PATENTSCHRIFT

(11) **EP 2 687 240 B1**
(45) Veröffentlichungstag und Bekanntmachung des Hinweises auf die Patenterteilung: **12.12.2018**
(21) Anmeldenummer: 13176535.6
(22) Anmeldetag: 15.07.2013
(51) Int. Cl.: A61L 27/02, A61L 27/12, A61L 24/02, A61L 27/28, A61L 27/50

(54) **Pastenförmiges Knochenersatzmaterial**
Paste-like bone substitute material
Matériau de remplacement d'os en forme de pâte

(30) Priorität: 20.07.2012 DE 102012014419
(43) Veröffentlichungstag der Anmeldung: 22.01.2014
(73) Patentinhaber: Heraeus Medical GmbH, 61273 Wehrheim (DE)
(72) Erfinder: Vogt, Sebastian, 99092 Erfurt (DE)
(74) Vertreter: Heraeus IP

(56) Entgegenhaltungen:
- EP-A1- 1 374 905
- EP-A2- 2 508 210

## Beschreibung

Die vorliegende Erfindung betrifft ein Verfahren zur Herstellung einer pastenförmigen Zusammensetzung zur Herstellung eines Knochenersatzmaterials.

Pastenförmige Knochenersatzmaterialien auf Basis von Wasser und in Wasser löslichen synthetischen Polymeren oder Biopolymeren sind seit langem bekannt. So wird beispielsweise in SU-A-1005344 ein pastenförmiges Knochenersatzmaterial vorgeschlagen, das aus Hydroxylapatit, Gelatine und Wasser zusammengesetzt ist.

Ein ähnliches pastöses Material wurde im EP-A-1 490 123 beschrieben, bei dem ein Hydrogel mit Keramik-Partikeln vollsynthetischen Ursprungs verwendet wird, welche eine unrunde Form aufweisen. Als Hydrogelbildner wird dabei bevorzugt Hyaluronsäure eingesetzt.

In WO-A-95/03074 wird ein pastöses Knochenersatzmaterial offenbart, das aus Wasser und einem darin suspendiertem Hydroxylapatit der Partikelgröße 0,015-0,06 µm zusammengesetzt ist.

In DE-A-10 2006 037 362 wird ein aus partikulärem Calciumcarbonat, Wasser und einem darin gelösten Hämostyptikum zusammengesetztes pastenförmiges Knochenersatzmaterial beschrieben.

Das Hämostyptikum ist dabei in einer solchen Menge in der Lösung enthalten, dass diese isotonisch ist.

Die nachveröffentlichte EP 2 508 210 A2 offenbart ein plastisch deformierbares Hämostyptikum, das zur Versiegelung von blutendem Knochengewebe einsetzbar ist. Das plastisch verformbare, biodegradierbare Hämostyptikum enthält wenigstens einen gesättigten Gycerin-1,2,3-trifettsäureester mit einer Schmelztemperatur von mehr als 37°C, einen Füllstoff mit einer Schmelztemperatur von mehr als 37°C und mindestens eine Verbindung mit einer Schmelztemperatur von nicht mehr als 37°C, die bei einer Temperatur von 25°C eine Löslichkeit von weniger als 50 Gramm pro Liter Wasser aufweist.

EP 1 374 905 A1 offenbart injizierbare Mikrodispersionen und pharmazeutische Zusammensetzungen, die ein flüssiges Polymer und ein Polymerwachs enthalten. Das flüssige Polymer hat einen Schmelzpunkt von weniger als 40°C und ist das Reaktionsprodukt einer mehrbasischen Säure oder eines ihrer Derivate, eines Polyols und einer Fettsäure.

Die aus dem Stand der Technik bekannten, wässrige Lösungen oder Hydrogele enthaltenden pastenförmigen Knochenersatzmaterialien haben jedoch den Nachteil, dass diese sehr häufig bei Kontakt mit wässrigen Lösungen, wie Wundsekret und Blut, innerhalb von Sekunden zerfallen beziehungsweise zerfließen. Diese Eigenschaft ist besonders störend, wenn in der Nähe des pastenförmigen Knochenersatzmaterials noch Reinigungsschritte mit Spülflüssigkeiten, wie physiologische Kochsalzlösung oder Ringer-Lösung, erforderlich sind.

Der vorliegenden Erfindung lag die Aufgabe zugrunde, die sich aus dem Stand der Technik ergebenden Nachteile im Zusammenhang mit pastenförmigen Knochenersatzmaterialien zu überwinden.

Das pastenförmige Knochenersatzmaterial soll manuell modellierbar sein und vorzugsweise eine Haftung auf Knochengewebe zeigen.

Das pastenförmige Knochenersatzmaterial soll im Vergleich zu den aus dem Stand der Technik bekannten Knochenersatzmaterialien durch eine verbesserte Stabilität gegenüber wässrigen Lösungen und Körperflüssigkeiten gekennzeichnet sein. Diese verbesserte Stabilität sollte sich insbesondere dadurch manifestieren, dass das pastenförmige Knochenersatzmaterial bei Kontakt mit diesen Flüssigkeiten nicht innerhalb weniger Minuten zerfällt.

Das pastenförmiges Knochenersatzmaterial soll im Vergleich zu den aus dem Stand der Technik bekannten Knochenersatzmaterialien bei einer Lagerung bei Raumtemperatur in geringerem Ausmaß zu einer Sedimentation neigen. Insbesondere soll das Material bei längerer Lagerung nicht in Partikel und einen flüssigen Überstand zerfallen.

Insbesondere lag der vorliegenden Erfindung die Aufgabe zugrunde, ein Verfahren anzugeben, mittels dessen pastenförmige Knochenersatzmaterialien mit den vorstehend beschriebenen Vorteilen auf möglichst einfache Art und Weise hergestellt werden können.

Einen Beitrag zur Lösung der eingangs genannten Aufgaben leistet ein Verfahren zur Herstellung einer pastenförmigen Zusammensetzung, mindestens beinhaltend die Verfahrensschritte:
(i) Bereitstellung von Partikeln mindestens eines Calciumsalzes;
(ii) zumindest teilweises, vorzugsweise vollständiges Beschichten der Partikel des mindestens einen Calciumsalzes mit einem gesättigten Fettsäureester (a), der eine Schmelztemperatur von mindestens 45°C, besonders bevorzugt von mehr als 55°C, aufweist und ausgewählt ist aus der Gruppe bestehend aus Glycerin-1,2,3-trimyristat, Glycerin-1,2,3-tripalmitat, Glycerin-1,2,3-tristearat, Glycerin-1,2,3- tribehenat, Myristinsäuremyristylester, Palmitinsäurepalmitoylester und einer Mischung aus mindestens zwei davon, und
(iii) Vermischen der zumindest teilweise, vorzugsweise vollständig mit dem gesättigten Fettsäureester (a) beschichteten Partikel des mindestens einen Calciumsalzes mit mindestens einem gesättigten Fettsäureester (b), der eine Schmelztemperatur von weniger als 25°C, besonders bevorzugt von weniger als 20°C, aufweist und ausgewählt ist aus der Gruppe bestehend aus Glycerin-1,2,3-trioctoat, Glycerin-1,2,3-tridecanoat, Myristinsäureisopropylester, Myristinsäuremethylester, Myristinsäureethylester, Palmitinsäureisopropylester, Palmitinsäuremethylester, Palmitinsäureethylester, Stearinsäureisoproyplester, Mischungen aus Triglyceriden von gesättigten Fettsäuren mit einer Kettenlänge in einem Bereich von 8 bis 12 Kohlenstoffatomen oder Mischungen aus mindestens zwei der vorstehend genannten Komponenten, unter Bildung einer pastenförmigen Zusammensetzung.

Die Komponenten werden in solchen relativen Mengen miteinander in Kontakt gebracht, dass eine pastenförmige Zusammensetzung erhalten wird, die
(α1) 50 bis 80 Gew.-%, besonders bevorzugt 55 bis 75 Gew.-% und am meisten bevorzugt 60 bis 70 Gew.-% der Partikel eines Caciumsalzes,
(α2) 0,1 bis 25 Gew.-%, besonders bevorzugt 1 bis 20 Gew.-% und am meisten bevorzugt 2,5 bis 12,5 Gew.-% des gesättigten Fettsäureesters (a),
(α3) 15 bis 40 Gew.-%, besonders bevorzugt 20 bis 35 Gew.-% und am meisten bevorzugt 22,5 bis 32,5 Gew.-% des gesättigten Fettsäureesters (b), und
(α4) 0 bis 10 Gew.-%, besonders bevorzugt 0,1 bis 8 Gew.-% und am meisten bevorzugt 1 bis 6 Gew.-% des Zusatzstoffes,
wobei die Summe der Komponenten (α1) bis (α4) 100 Gew.-% beträgt.

Das zumindest teilweise, vorzugsweise das vollständige Beschichten der Partikel des mindestens einen Calciumsalzes mit einem gesättigten Fettsäureester (a) im Verfahrensschritt ii) kann auf verschiedene Art und Weise erfolgen. So können beispielsweise zunächst die Partikel des Calciumsalzes mit dem gesättigten Fettsäureester (a) in festem Zustand (i. e. bei einer Temperatur unterhalb von 45 °C) vermischt, anschließend die Mischung bei einer Temperatur oberhalb von 45 °C, beispielsweise bei einer Temperatur von mindestens 60°C oder mindestens 90°C für eine Dauer von vorzugsweise mindestens 1 Stunde, besonders bevorzugt mindestens 2 Stunden, vorzugsweise unter Mischbedingungen getempert und anschließend vor der Zugabe des gesättigten Fettsäureesters (b) im Verfahrensschritt (iii) gegebenenfalls wieder auf eine Temperatur unterhalb von 45 °C abgekühlt werden. Denkbar ist auch, zunächst den gesättigten Fettsäureester (a) auf eine Temperatur oberhalb des Schmelzpunktes (i. e. 45°C) zu erhitzen und dann den gesättigten Fettsäureester (a) in flüssigem Zustand mit den Partikeln des Calciumsalzes zu vermischen.

Gemäß einer besonderen Ausgestaltung des erfindungsgemäßen Verfahrens beinhaltet dieses weiterhin den Verfahrensschritt
(iv) Hinzufügen mindestens eines Zusatzstoffes zu den Partikeln des Calciumsalzes, dem gesättigten Fettsäureester (a), dem gesättigten Fettsäureester (b) oder einer Mischung aus mindestens zwei dieser Komponenten,
wobei als Zusatzstoff wiederum diejenigen Verbindungen bevorzugt sind, die bereits eingangs im Zusammenhang mit der erfindungsgemäßen pastenförmigen Zusammensetzung als bevorzugte Zusatzstoffe genannt wurden.
beinhaltet, wobei die Summe der Komponenten (α1) bis (α4) 100 Gew.-% beträgt.

Erfindungsgemäß besonders bevorzugt ist es, wenn der mindestens eine Zusatzstoffe nach dem mindestens teilweisen, vorzugsweide dem vollständigen Beschichten der Partikel des Calciumsalzes mit dem gesättigten Fettsäureester (a) gemäß Verfahrensschritt ii) und vor dem Vermischen mit dem gesättigten Fettsäureester (b) gemäß Verfahrensschritt iii) hinzugefügt wird.

Die hergestellte pastenförmige Zusammensetzung enthält vorzugsweise nicht hydraulisch abbindende Partikel mindestens eines Calciumsalzes, wobei die Partikel des mindestens einen Calciumsalzes
- mit mindestens einem gesättigten Fettsäureester (a), der eine Schmelztemperatur von mindestens 45°C, besonders bevorzugt von mindestens 55°C aufweist, und ausgewählt ist aus der Gruppe bestehend aus Glycerin-1,2,3-trimyristat, Glycerin-1,2,3-tripalmitat, Glycerin-1,2,3-tristearat, Glycerin-1,2,3-tribehenat, Myristinsäuremyristylester, Palmitinsäurepalmitoylester und einer Mischung aus mindestens zwei davon,
   mindestens teilweise, vorzugsweise vollständig, beschichtet sind, und
- diese zumindest teilweise, vorzugsweise vollständig mit dem gesättigten Fettsäureester (a) beschichteten Partikel mit mindestens einem gesättigten Fettsäureester (b), der eine Schmelztemperatur von weniger als 25°C, besonders bevorzugt von weniger als 20°C aufweist, wobei der gesättigte Fettsäureester (b) ganz besonders bevorzugt bei Raumtemperatur (i. e. 20 °C) flüssig ist, und ausgewählt ist aus der Gruppe bestehend aus Glycerin-1,2,3-trioctoat, Glycerin-1,2,3-tridecanoat, Myristinsäureisopropylester, Myristinsäuremethylester, Myristinsäureethylester, Palmitinsäureisopropylester, Palmitinsäuremethylester, Palmitinsäureethylester, Stearinsäureisoproyplester, Mischungen aus Triglyceriden von gesättigten Fettsäuren mit einer Kettenlänge in einem Bereich von 8 bis 12 Kohlenstoffatomen oder Mischungen aus mindestens zwei der vorstehend genannten Komponenten,
unter Bildung einer pastenförmigen Zusammensetzung vermischt sind, wobei die Zusammensetzung
(α1) 50 bis 80 Gew.-% der Partikel eines Calciumsalzes,
(α2) 0,1 bis 25 Gew.-% des gesättigten Fettsäureesters (a),
(α3) 15 bis 40 Gew.-% des gesättigten Fettsäureesters (b), und
(α4) 0 bis 10 Gew.-% eines Zusatzstoffes
beinhaltet, wobei die Summe der Komponenten (α1) bis (α4) 100 Gew.-% beträgt.

Die Erfindung basiert auf der überraschenden Beobachtung, dass aus mit gesättigten Fettsäureestern (a) mindestens teilweise, vorzugsweise vollständig beschichtete Partikel von Calciumsalzen beim Vermischen mit bei Raumtemperatur flüssigen gesättigten Fettsäureestern (b) Pasten erhalten werden können, die modellierbar sind und die im Gegensatz zu Gemischen aus unbeschichteten Partikeln von Calciumsalzen und bei Raumtemperatur flüssigen gesättigten Fettsäureestern nicht sedimentieren. Das bedeutet, dass die Paste bei einer Lagerung nicht in Partikel und flüssigen Überstand zerfällt. Weiterhin zeigt die erfindungsgemäße pastenförmige Zusammensetzung eine gute Haftwirkung sowohl auf feuchten als auch auf fettigen Oberflächen, wie Knochengewebe.

Bei den Partikel des Calciumsalzes handelt es sich vorzugsweise um Partikel ausgewählt aus der Gruppe bestehend aus β-Tricalciumphosphat, α-Tricalcium-phosphat, Hydroxylapatit, Octacalciumphosphat, Calciumcarbonat, Dolomit, Calciumsulfat-Dihydrat und Mischungen aus mindestens zwei davon. Besonders bevorzugt zeichnen sich die Partikel des Calciumsalzes durch ihre Eigenschaft aus, bei Einwirkung von Wasser keine Verfestigungsreaktion zu zeigen. Das bedeutet, dass die erfindungsgemäße pastenförmige Zusammensetzung nach Kontakt mit Wasser oder wässrigen Lösungen nicht im Sinne einer hydraulischen Abbindereaktion wie bei Calciumphosphatzementen und bei Calciumsulfatzementen reagiert (i. e. die Partikel des Calciumsalzes sind vorzugsweise nicht hydraulisch abbindend). Übliche Calciumphosphatzemente basieren auf amorphen Calcium-phosphat, Tricalciumphosphaten oder auch Tetracalciumphosphat. Die Aushärtung von Calciumsulfatzementen beruht auf der Anlagerung von Wasser an Calciumsulfat-Hemihydrat, wobei sich Calciumsulfat-Dihydrat bildet.

Im Zusammenhang mit den Partikeln des Calciumsalzes ist es insbesondere bevorzugt, dass diese zu mindestens 50 Gew.-%, besonders bevorzugt zu mindestens 75 Gew.-%, noch mehr bevorzugt zu mindestens 95 Gew.-% und am meisten bevorzugt zu 100 Gew.-%, jeweils bestimmt durch Siebanalyse, eine Partikelgröße von weniger als 1 mm, besonders bevorzugt von weniger als 100 µm und noch mehr bevorzugt von weniger als 64 µm aufweisen.

Bei dem gesättigten Fettsäureester (a) handelt es sich um einen Fettsäureester ausgewählt aus der Gruppe bestehend aus Glycerin-1,2,3-trimyristat, Glycerin-1,2,3-tripalmitat, Glycerin-1,2,3-tristearat, Glycerin-1,2,3-tribehenat, Myristinsäuremyristylester, Palmitinsäurepalmitoylester und einer Mischung aus mindestens zwei davon.

Bei dem gesättigten Fettsäureester (b) handelt es sich um einen Fettsäureester ausgewählt aus der Gruppe bestehend aus Glycerin-1,2,3-trioctoat, Glycerin-1,2,3-tridecanoat, Myristinsäureisopropylester, Myristinsäuremethylester, Myristinsäureethylester, Palmitinsäureisopropylester, Palmitinsäuremethylester, Palmitinsäureethylester, Stearinsäureisoproyplester, Mischungen aus Triglyceriden von Fettsäuren mit einer Kettenlänge in einem Bereich von 8 bis 12 Kohlenstoffatomen oder Mischungen aus mindestens zwei der vorstehend genannten Komponenten. Bevorzugte Mischungen aus Triglyceriden von gesättigten Fettsäuren mit einer Kettenlänge in einem Bereich von 8 bis 12 Kohlenstoffatomen sind beispielsweise die unter dem Handelsnamen MYGLIOL® oder ESTASAN® erhältlichen Mischungen. Beim MYGLIOL® handelt es sich um gemischte, gesättigte Decanoyl- und Octanoylester des Glycerins (CAS 52622-27-2). Die gesättigten Fettsäureester (b) zeichnen sich vorzugsweise dadurch aus, das sie bei Raumtemperatur (i. e. 20°C) flüssig sind.

Gemäß einer besonderen Ausgestaltung der erfindungsgemäßen pastenförmigen Zusammensetzung beinhaltet diese neben den Partikeln eines Calciumsalzes und den gesättigten Fettsäureestern (a) und (b) weitere Zusatzstoffe, wobei als Zusatzstoffe insbesondere pharmazeutische Wirkstoffe in Betracht kommen, die in der pastenförmigen Zusammensetzung in gelöster oder suspendierter Form enthalten sein können.

Der pharmazeutische Wirkstoff kann vorzugsweise aus der Gruppe ausgewählt sein, die aus Antibiotika, Aniphlogistika, Steroiden, Hormonen, Wachstumsfaktoren, Bisphosphonaten, Cytostatika und Genvektoren besteht. Gemäß einer besonders bevorzugten Ausführungsform handelt es sich bei dem wenigstens einen pharmazeutischen Wirkstoff um ein Antibiotikum. Das wenigstens eine Antibiotikum ist vorzugsweise aus der Gruppe ausgewählt, die aus Aminoglykosid-Antibiotika, Glykopeptid-Antibiotika, Lincosamid-Antibiotika, Gyrasehemmern, Carbapenemen, cyclischen Lipopeptiden, Glycylcyclinen, Oxazolidonen und Polypeptidantibiotika besteht.

Gemäß einer besonders bevorzugten Ausführungsform handelt es sich bei dem wenigstens einen Antibiotikum um ein Mitglied, das aus der Gruppe ausgewählt ist, die aus Gentamicin, Tobramycin, Amikacin, Vancomycin, Teicoplanin, Dalbavancin, Lincosamin, Clindamycin, Moxifloxacin, Levofloxacin, Ofloxacin, Ciprofloxacin, Doripenem, Meropenem, Tigecyclin, Linezolid, Eperezolid, Ramoplanin, Metronidazol, Tinidazol, Omidazol und Colistin sowie Salzen und Estern davon besteht.

Demnach kann das wenigstens eine Antibiotikum aus der Gruppe ausgewählt sein, die aus Gentamicinsulfat, Gentamicinhydrochlorid, Amikacinsulfat, Amikacinhydrochlorid, Tobramycinsulfat, Tobramycinhydrochlorid, Clindamycin-hydrochlorid, Lincosaminhydrochlorid und Moxifloxacin besteht.

Das wenigstens eine Antiphlogistikum ist vorzugsweise aus der Gruppe ausgewählt, die aus nicht-steroidalen Antiphlogistika und Glukokortikoiden besteht. Gemäß einer besonders bevorzugten Ausführungsform ist das wenigstens eine Antiphlogistikum aus der Gruppe ausgewählt, die aus Acetylsalicylsäure, Ibuprofen, Diclofenac, Ketoprofen, Dexamethason, Prednison, Hydrocortison, Hydrocortisonacetat und Fluticason besteht.

Das wenigstens eine Hormon ist vorzugsweise aus der Gruppe ausgewählt, die aus Serotonin, Somatotropin, Testosteron und Östrogen besteht.

Der wenigstens eine Wachstumsfaktor ist vorzugsweise aus der Gruppe ausgewählt, die aus dem *Fibroblast Growth Factor* (FGF), dem *Transforming Growth Factor* (TGF), dem *Platelet Derived Growth Factor* (PDGF), dem Epidermalen Wachstumsfaktor (EGF), dem *Vascular Endothelial Growth Factor* (VEGF), insulinähnlichen Wachstumsfaktoren (IGF), dem *Hepatocyte Growth Factor* (HGF), dem *Bone Morphogenetic Protein* (BMP), Interleukin-1B, Interleukin 8 und dem Nervenwachstumsfaktor besteht.

Das wenigstens eine Cytostatikum ist vorzugsweise aus der Gruppe ausgewählt, die aus Alkylantien, Platinanaloga, Interkalantien, Mitosehemmern, Taxanen, Topoisomerasehemmern und Antimetaboliten besteht.

Das wenigstens eine Bisphosphonat ist vorzugsweise aus der Gruppe ausgewählt, die aus Zoledronat und Aledronat besteht.

### Weiterhin beinhaltet die pastenförmige Zusammensetzung

(α1) 50 bis 80 Gew.-%, besonders bevorzugt 55 bis 75 Gew.-% und am meisten bevorzugt 60 bis 70 Gew.-% der Partikel eines Calciumsalzes,
(α2) 0,1 bis 25 Gew.-%, besonders bevorzugt 1 bis 20 Gew.-% und am meisten bevorzugt 2,5 bis 12,5 Gew.-% des gesättigten Fettsäureesters (a),
(α3) 15 bis 40 Gew.-%, besonders bevorzugt 20 bis 35 Gew.-% und am meisten bevorzugt 22,5 bis 32,5 Gew.-% des gesättigten Fettsäureesters (b), und
(α4) 0 bis 10 Gew.-%, besonders bevorzugt 0,1 bis 8 Gew.-% und am meisten bevorzugt 1 bis 6 Gew.-% des Zusatzstoffes,
wobei die Summe der Komponenten (α1) bis (α4) 100 Gew.-% beträgt.

Die Erfindung wird durch die nachstehend beschriebenen Beispiele näher erläutert, die jedoch die Erfindung nicht einschränken.

### AUSFÜHRUNGSBEISPIELE

### Beispiel 1

Ein Gemisch von 23,60 g Calciumsulfat-Dihydrat, 5,90 g Calciumcarbonat und 0,50 g Gentamicinsulfat (AK 600) werden zusammen mit 3 Prozellankugeln in eine Plastikflasche eingewogen. Danach wird das Gemisch mit Hilfe eines Turbula-Mischers 30 Minuten gemischt. 20,00 g des gemahlenen Gemisch werden in einem Becherglas mit 1,98 g Glycerin-1,2,3-tripalmitat vermischt. Dieses Gemisch wird 5 Stunden bei 90°C unter gelegentlichem Rühren getempert. Danach wird das Gemisch mit 7,33 g MYGLIOL® 812 verknetet. Es entsteht eine homogene, farblose Paste.

### Beispiel 2

Es werden 20,00 g Calciumcarbonat in einem Becherglas mit 3,00 g Glycerin-1,2,3-tripalmitat vermischt. Dieses Gemisch wird 5 Stunden bei 90°C unter gelegentlichem Rühren getempert. Danach wird das Gemisch mit 8,94 g Glycerin-1,23-trioctoat verknetet. Es entsteht eine farblose Paste.

### Beispiel 3

Es werden 20,00 g Tricalciumphosphat in einem Becherglas mit 4,00 g Glycerin-1,2,3-tristearat vermischt. Dieses Gemisch wird 5 Stunden bei 90°C unter gelegentlichem Rühren getempert. Danach wird das Gemisch mit 7,58 g Glycerin-1,23-trioctoat verknetet. Es entsteht eine farblose Paste.

Die Pasten der Beispiele 1-3 ließen sich problemlos mit der Hand kneten und formen.

Es wurden dann jeweils 10 g der Pasten in 100 ml entionisiertes Wasser gegeben. Die Pastenproben blieben über einen Beobachtungszeitraum von 30 Minuten bei Raumtemperatur vollständig stabil und zersetzten sich nicht.

Die Pasten der Beispiele 1-3 zeigten bei Raumtemperatur über einen Zeitraum von 2 Monaten keine Sedimentation.

## Patentansprüche

1. Verfahren zur Herstellung einer pastenförmigen Zusammensetzung zur Herstellung eines Knochenersatzmaterials, mindestens beinhaltend die Verfahrensschritte:
(i) Bereitstellung von Partikeln mindestens eines Calciumsalzes;
(ii) zumindest teilweises Beschichten der Partikel des mindestens einen Calciumsalzes mit einem gesättigten Fettsäureester (a), der eine Schmelztemperatur von mindestens 45°C aufweist und ausgewählt ist aus der Gruppe bestehend aus Glycerin-1,2,3-trimyristat, Glycerin-1,2,3-tripalmitat, Glycerin-1,2,3-tristearat, Glycerin-1,2,3-tribehenat, Myristinsäuremyristylester, Palmitinsäurepalmitoylester und einer Mischung aus mindestens zwei davon und
(iii) Vermischen der zumindest teilweise mit dem gesättigten Fettsäureester (a) beschichteten Partikel des mindestens einen Calciumsalzes mit mindestens einem gesättigten Fettsäureester (b), der eine Schmelztemperatur von weniger als 25°C aufweist und ausgewählt ist aus der Gruppe bestehend aus Glycerin-1,2,3-trioctoat, Glycerin-1,2,3-tridecanoat, Myristinsäureisopropylester, Myristinsäuremethylester, Myristinsäureethylester, Palmitinsäureisopropylester, Palmitinsäuremethylester, Palmitinsäureethylester, Stearinsäureisoproyplester, Mischungen aus Triglyceriden von gesättigten Fettsäuren mit einer Kettenlänge in einem Bereich von 8 bis 12 Kohlenstoffatomen oder Mischungen aus mindestens zwei der vorstehend genannten Komponenten, unter Bildung einer pastenförmigen Zusammensetzung,
wobei die Komponenten in solchen relativen Mengen miteinander in Kontakt gebracht werden, dass eine pastenförmige Zusammensetzung erhalten wird, die
(α1) 50 bis 80 Gew.-% der Partikel eines Cacliumsalzes,
(α2) 0,1 bis 25 Gew.-% des gesättigten Fettsäureesters (a),
(α3) 15 bis 40 Gew.-% des gesättigten Fettsäureesters (b), und
(α4) 0 bis 10 Gew.-% eines Zusatzstoffes
beinhaltet, wobei die Summe der Komponenten (α1) bis (α4) 100 Gew.-% beträgt.

2. Verfahren nach Anspruch 1, wobei die Partikel des Calciumsalzes ausgewählt sind aus der Gruppe bestehend aus β-Tricalciumphosphat, α-Tricalciumphosphat, Hydroxylapatit, Octacalciumphosphat, Calciumcarbonat, Dolomit, Calciumsulfat-Dihydrat und Mischungen aus mindestens zwei davon.

3. Verfahren nach Anspruch 1 oder 2, wobei mindestens 50 Gew.-% der Partikel des Calciumsalzes eine durch Siebanalyse bestimmte Partikelgröße von weniger als 1 mm aufweisen.

4. Verfahren nach einem der Ansprüche 1 bis 3, wobei mindestens 50 Gew.-% der Partikel des Calciumsalzes eine durch Siebanalyse bestimmte Partikelgröße von weniger als 100 µm aufweisen.

5. Verfahren nach einem der Ansprüche 1 bis 4, wobei mindestens 50 Gew.-% der Partikel des Calciumsalzes eine durch Siebanalyse bestimmte Partikelgröße von weniger als 64 µm aufweisen.

6. Verfahren nach einem der Ansprüche 1 bis 5, weiterhin beinhalten den Verfahrensschritt (iv) Hinzufügen mindestens eines Zusatzstoffes zu den Partikeln des Calciumsalzes, dem gesättigten Fettsäureester (a), dem gesättigten Fettsäureester (b) oder einer Mischung aus mindestens zwei dieser Komponenten.

## Claims

1. Method for the production of a pasty composition for production of a bone replacement material, comprising at least the procedural steps of:
(i) providing particles of at least one calcium salt;
(ii) coating, at least partially, the particles of the at least one calcium salt with a saturated fatty acid ester (a) that has a melting temperature of at least 45°C and is selected from the group consisting of glycerol-1,2,3-trimyristate, glycerol-1,2,3-tripalmitate, glycerol-1,2,3-tristearate, glycerol-1,2,3-tribehenate, myristic acid myristyl ester, palmitic acid palmitoyl ester, and a mixture of at least two thereof; and
(iii) mixing the particles of the at least one calcium salt, which are coated, at least partially, with saturated fatty acid ester (a), and at least one saturated fatty acid ester (b), which has a melting temperature of less than 25°C and is selected from the group consisting of glycerol-1,2,3-trioctoate, glycerol-1,2,3-tridecanoate, myristic acid isopropyl ester, myristic acid methyl ester, myristic acid ethyl ester, palmitic acid isopropyl ester, palmitic acid methyl ester, palmitic acid ethyl ester, stearic acid isopropyl ester, mixtures of triglycerides of saturated fatty acids with a chain length in a range of 8 to 12 carbon atoms or mixtures of at least two of the components specified above, while forming a pasty composition,
whereby the components are contacted to each other in appropriate relative amounts such that a pasty composition is obtained, which contains
(α1) 50 to 80% by weight of the particles of a calcium salt;
(α2) 0.1 to 25% by weight of saturated fatty acid ester (a);
(α3) 15 to 40% by weight of saturated fatty acid ester (b); and
(α4) 0 to 10% by weight of an additive,
whereby the sum of components (α1) to (α4) is 100% by weight.

2. Method according to claim 1, whereby the particles of the calcium salt are selected from the group consisting of β-tricalcium phosphate, α-tricalcium phosphate, hydroxylapatite, octacalcium phosphate, calcium carbonate, dolomite, calcium sulfate dihydrate, and mixtures of at least two thereof.

3. Method according to claim 1 or 2, whereby at least 50% by weight of the particles of the calcium salt have a particle size of less than 1 mm, as determined by sieve analysis.

4. Method according to any one of the claims 1 to 3, whereby at least 50% by weight of the particles of the calcium salt have a particle size of less than 100 µm, as determined by sieve analysis.

5. Method according to any one of the claims 1 to 4, whereby at least 50% by weight of the particles of the calcium salt have a particle size of less than 64 µm, as determined by sieve analysis.

6. Method according to any one of the claims 1 to 5, further comprising procedural step (iv), adding at least one additive to the particles of the calcium salt, saturated fatty acid ester (a), saturated fatty acid ester (b) or to a mixture of at least two of said components.

## Revendications

1. Procédé de fabrication d'une composition pâteuse pour la fabrication d'un matériau de remplacement osseux, contenant au moins les étapes de procédé :
(i) mise à disposition de particules d'au moins un sel de calcium ;
(ii) enrobage au moins partiel des particules de l'au moins un sel de calcium avec un ester d'acide gras saturé (a) qui présente une température de fusion d'au moins 45°C et est sélectionné parmi le groupe constitué du 1,2,3-trimyristate de glycérine, 1,2,3-tripalmitate de glycérine, 1,2,3-tristéarate de glycérine, 1,2,3-tribéhénate de glycérine, ester myristylique d'acide myristique, ester palmitoylique d'acide palmitique et d'un mélange d'au moins deux de ceux-ci, et
(iii) mélange des particules de l'au moins un sel de calcium enrobées au moins partiellement avec l'ester d'acide gras saturé (a) avec au moins un ester d'acide gras saturé (b) qui présente une température de fusion de moins de 25°C et est sélectionné parmi le groupe constitué du 1,2,3-trioctoate de glycérine, 1,2,3-tridécanoate de glycérine, ester isopropylique d'acide myristique, ester méthylique d'acide myristique, ester éthylique d'acide myristique, ester isopropylique d'acide palmitique, ester méthylique d'acide palmitique, ester éthylique d'acide palmitique, ester isopropylique d'acide stéarique, de mélanges de triglycérides d'acides gras saturés avec une longueur de chaîne dans une plage de 8 à 12 atomes de carbone ou de mélanges d'au moins deux des composants cités précédemment, avec formation d'une composition pâteuse,
dans lequel les composants sont amenés en contact les uns avec les autres en des quantités relatives telles qu'une composition pâteuse est obtenue, laquelle contient
(α1) 50 à 80 % en poids des particules d'un sel de calcium,
(α2) 0,1 à 25 % en poids de l'ester d'acide gras saturé (a),
(α3) 15 à 40 % en poids de l'ester d'acide gras saturé (b), et
(α4) 0 à 10 % en poids d'un additif,
dans lequel la somme des composants (α1) à (α4) se monte à 100 % en poids.

2. Procédé selon la revendication 1, dans lequel les particules du sel de calcium sont sélectionnées parmi le groupe constitué du β-phosphate tricalcique, α-phosphate tricalcique, hydroxylapatite, phosphate octacalcique, carbonate de calcium, dolomite, sulfate de calcium dihydraté et de mélanges d'au moins deux de ceux-ci.

3. Procédé selon la revendication 1 ou 2, dans lequel au moins 50 % en poids des particules du sel de calcium présentent une taille particulaire déterminée par analyse granulométrique de moins de 1 mm.

4. Procédé selon une des revendications 1 à 3, dans lequel au moins 50 % en poids des particules du sel de calcium présentent une taille particulaire déterminée par analyse granulométrique de moins de 100 µm.

5. Procédé selon une des revendications 1 à 4, dans lequel au moins 50 % en poids des particules du sel de calcium présentent une taille particulaire déterminée par analyse granulométrique de moins de 64 µm.

6. Procédé selon une des revendications 1 à 5, contenant en outre l'étape de procédé (iv) ajouter au moins un additif aux particules du sel de calcium, à l'ester d'acide gras saturé (a), à l'ester d'acide gras saturé (b) ou à un mélange d'au moins deux de ces composants.
